# EUROPEAN PATENT SPECIFICATION

(11) **EP 0 699 758 B1**
(45) Date of publication and mention of the grant of the patent: **24.07.2002**
(21) Application number: 95100871.3
(22) Date of filing: 12.12.1989
(51) Int. Cl.: C12N 7/00, C12N 15/48, A61K 39/21

(54) **Prototype felv isolates for use in disease models and vaccines**
Prototypische FeLV-Isolate für die Verwendung als Krankheitsmuster oder Impfstoff
Isolats FeLV prototypes, pour utilisation comme modèles de maladie, ou comme vaccins

(30) Priority: 13.12.1988 US 284139
(43) Date of publication of application: 06.03.1996
(62) Divisional of application: 89122964.3
(73) Proprietor: PRESIDENT AND FELLOWS OF HARVARD COLLEGE, Cambridge, Massachusetts 02138 (US); COLORADO STATE UNIVERSITY RESEARCH FOUNDATION, Fort Collins Colorado 80521 (US)
(72) Inventor: Hoover, Edward A., Fort Collins, Colorado (US); Mullins, James I., Seattle, Washington 98105 - 5333 (US)
(74) Representative: Gowshall, Jonathan Vallance

(56) References cited:
- EP-A- 0 262 887
- SCIENCE, vol. 239, 1988 pages 906-910, J. OVERBAUGH ET AL. 'Molecular cloning of a feline leukemia virus that induces fatal immunodeficiency disease in cats'
- J. VIROL., vol. 62, no. 2, 1988 pages 722-731, P. DONAHUE ET AL, 'Strong sequence conservation among horizontally transmissible minimally pathogenic feline leukemia viruses'
- VET. IMMUNOL. IMMUNOPATHOL., vol. 11, 1986 pages 123-148, N. PESERSEN ET AL. 'Possible enhancement of persistent viremia by feline leukemia envelope glycoprotein vaccines in challenge-exposure situations where whole inactivated virus vaccines were protective'
- J. VIROL., vol. 58, no. 3, 1986 pages 825-834, M. STEWART ET AL. 'Nucleotide sequences of a feline leukemia virus subgroup A envelope gene and long terminal repeat and evidence for the recombinational origin of subtype B viruses'

## Description

### Technical Field

The present invention is generally directed toward the derivation of molecular clones of FeLV and their use as vaccines and in disease models.

### Background of the Invention

Retroviruses form a large class of enveloped RNA viruses which invade a large number of specific mammalian hosts. They are infectiously transmitted by a variety of mechanisms, are frequently associated with severe diseases, and share common elements among their structures. The retroviruses, consisting of a (+) strand RNA dimer (ssRNA), form long terminal repeats ("LTR") in their proviral DNA intermediates and a genome coding for capsid proteins ("the gag gene"), reverse transcriptase and integrase functions (the "pol" gene), and the membrane envelope gene ("env"). With particular viruses, other open reading frames have been shown to be present coding for proteins having specific functions in addition to the functions of the common genes.

Feline leukemia viruses (FeLV) are exogenous type C retroviruses that are responsible for induction of a diverse series of lymphoreticular diseases of outbred cats including lymphosarcoma, leukemia, aplastic anemia, myelodysplasia, and feline acquired immunodeficiency syndrome (Hardy et al., Cancer Res. 36:582, 1976; Hardy, Feline Leukemia Virus, Hardy, Essex, McCelland, eds. (Elsevier/North Holland, 1980), pp. 3-31; Hoover, Rojko, Olsen, Feline Leukemia, Olsen, ed. (CRC Press, Boca Raton, Fla., 1980), pp. 32-51; Hardy and Essex, Prog. Allergy 37:353, 1986). The genome of FeLV is a 60-70S dimer of single-stranded RNA consisting of a gag gene encoding the capsid proteins, a pol gene encoding the protease, reverse transcriptase and integrase, and an env gene encoding the gp70 and p15E viral envelope proteins. As with other retroviruses, when a susceptible cell is infected with FeLV (in vivo or in vitro), the genome is transcribed into a double-stranded DNA copy which is then carried in the cellular DNA as a provirus organized into 5'-LTR (long terminal repeat)-gag-pol-env-LTR-3' regions. The integrated provirus then serves as the template for production of FeLV RNAs and ultimately infectious virions.

FeLV have been classified into subgroups A, B, and C on the basis of virus interference and neutralization assays, and the distribution of the subgroups within feline populations differs markedly. Subgroup A viruses have been found in all naturally occurring infections examined, either alone or in combination with B and C. Subgroup B, found in approximately 40% of all infections, and subgroup C, found in perhaps 1% of infections, occur as mixed infections of subgroups A and B, A and C, or A, B, and C. Subgroup identity also correlates with the host range of infection in vitro and pathogenicity in cats: FeLV-A isolates are sometimes restricted to growth in feline cells and are minimally pathogenic; FeLV-B isolates grow in heterologous cells such as human and canine fibroblastoid cells and are found at a higher frequency in cats with proliferative disease; and the rare FeLV-C isolates have an extended host range including human, canine, and guinea pig cells and are capable of inducing aplastic anemia.

In view of the devastating effect of FeLV-induced disease in domestic cats, the prevention of FeLV infection through vaccination of susceptible animals is a high priority for veterinary researchers. Numerous attempts to produce such a vaccine have been largely unsuccessful and, in fact, the only commercially available vaccine (Leukocell™, Norden Laboratories, Lincoln, Nebraska) has been severely criticized for its questionable efficacy (Pedersen et al., Feline Practice 15:7-20, 1985). The present invention fulfills the need in the art for a suitable FeLV vaccine, and further provides other related advantages, including the definition of a prototype highly infectious FeLV-A challenge virus, a prototype molecularly cloned immunodeficiency inducing FeLV, and a disease model useful for the study of retrovirus-induced immunodeficiency syndrome and leukemia in cats and humans.

### Disclosure of Invention

Briefly stated, the present invention discloses molecular clones of feline leukemia virus isolates that encode (a) a prototype highly infectious, minimally pathogenic virus, (b) a variant genome that is replication-defective and associated with a fatal immunodeficiency in cats similar to AIDS (Hoover, Blood 70: 1880-1892, 1987; Overbaugh et al., Science 239:906-910, 1988), and (c) a chimeric genome that is replication-competent and induces the immunodeficiency syndrome described above. More specifically, these molecular clones are used to generate cell lines producing infectious virus which is useful in the preparation of vaccines or the generation of viremia or disease challenge systems.

In one aspect of the present invention, an isolated DNA sequence encoding the proviral genome of a FeLV-A subtype or a biological derivative thereof is disclosed. For purposes of the present invention, FeLV-A subtype or "biological derivative thereof" includes mutants of an FeLV-A subtype which are at least 92% homologous to a FeLV-A subtype. Within certain embodiments, the DNA sequence may be derived from proviral molecular clone 61C, which is replication-defective and not capable of inducing viremia in feline species (Felis domestica), or clones 61E or EECC, which are capable of inducing persistent viremia in feline species.

Recombinant plasmids capable of directing the expression of the proviral genome of a FeLV-A subtype or biological derivative thereof, and mammalian cells transfected with such a recombinant plasmid are also disclosed. Suitable mammalian cells include feline cells, such as AH927, CRFK, FCWF, Fc9, feline embryo fibroblasts or primary feline cell cultures, and mink lung cells.

Within another aspect of the present invention, a method of producing a FeLV-A subtype or biological derivative thereof is disclosed. The method generally comprises: (a) transfecting a mammalian host cell with a recombinant plasmid capable of directing the expression of the proviral genome of a FeLV-A subtype or biological derivative thereof, the plasmid comprising a DNA sequence encoding the proviral genome of a FeLV-A subtype or biological derivative thereof; (b) growing the host cell in an appropriate medium: and (c) separating the FeLV-A subtype or biological derivative thereof from the host cells. Suitable DNA sequences include those derived from clones 61E and EECC. Within an alternative embodiment, the mammalian host cell is cotransfected with a recombinant plasmid as briefly described above, the plasmid comprising a DNA sequence derived from the clone 61C and a DNA sequence encoding a replication-competent proviral genome, such as a DNA sequence derived from the clone 61E. The methods may also include, after the step of separating, purifying the FeLV-A subtype or biological derivative thereof by methods well known in the art.

Within a related aspect of the present invention, a method of producing a FeLV vaccine is disclosed. The method generally comprises: (a) transfecting a mammalian host cell with a recombinant plasmid capable of directing the expression of the proviral genome of a FeLV-A subtype or biological derivative thereof; (b) growing the host cell in an appropriate medium; (c) harvesting the FeLV-A subtype or biological derivative immunogens; and (d) inactivating the FeLV-A subtype or biological derivative thereof. Within an alternative embodiment, the mammalian host cell is cotrasfected as described above. The FeLV-A subtype or biological derivative thereof may be inactivated in a number of ways, including exposure to formalin, beta-propriolactone or binary ethyleneimine (BEI) under conditions and for a time sufficient to cause inactivation. The method may also include, after the step of inactivating, concentrating the FeLV-A subtype to achieve an antigenic mass suitable for clinical administration to provide protection in the feline species.

The present invention also discloses methods for protecting a feline host from FeLV infection. In one such method, an immunogenically effective amount of a composition comprising an inactivated FeLV-A subtype or biological derivative thereof in combination with a physiologically acceptable carrier or diluent is administered to the feline host. Suitable carriers or diluents include sterile water and phosphate buffered saline. The composition may also include a suitable adjuvant, such as complete or incomplete Freund's adjuvant, RIBI, oil adjuvants, particulate adjuvants such as aluminum hydroxide and aluminum phosphate, or general immune stimulating adjuvants, such as avridine and ama 31-91. In one aspect of the present invention, the physiologically acceptable carrier or diluent comprises cell-free supernatant derived from FeLV-A subtype-producing cells. In this regard, the physiologically acceptable carrier or diluent may further include whole cell lysates of FeLV-A subtype-producing cells. Within a related method, an immunogenically effective amount of a composition comprising the env gene product of a FeLV-A subtype is administered in combination with a physiologically acceptable carrier or diluent.

As noted above, the present invention discloses vaccines against FeLV-induced disease. In one aspect of the present invention, the vaccine comprises an inactivated FeLV-A subtype or biological derivative thereof in combination with a suitable adjuvant. Within preferred embodiments, the FeLV-A subtype or biological derivative thereof is encoded by a DNA sequence derived from clone EECC or clone 61E.

Within another aspect of the present invention, the vaccine comprises a FeLV-A subtype encoded by a DNA sequence derived from a FeLV clone which is replication competent, such as clone 61E, in combination with a biological derivative of a FeLV-A subtype encoded by a DNA sequence derived from clone 61C, along with a physiologically acceptable carrier or diluent.

In yet another aspect of the present invention, feline hosts exhibiting fatal immunodeficiency disease induced by inoculation with a FeLV-A subtype or biological derivative thereof are disclosed. These feline hosts are useful as disease models for both cats and other mammals, including humans.

These and other aspects of the present invention will become evident upon reference to the following detailed description and attached drawings.

### Brief Description of the Drawings

Figure 1 is a depiction of the complete nucleotide sequence of clone 61E. The sequence begins at the 5'LTR and extends to the 3'LTR corresponding to the 8440 nucleotide region shown schematically in Figure 2. Deduced amino acid sequences of the two major open reading frames (ORFs) are designated by a single letter code beneath the DNA sequence. Restriction sites also noted in Figure 2 are shown above the DNA sequence.

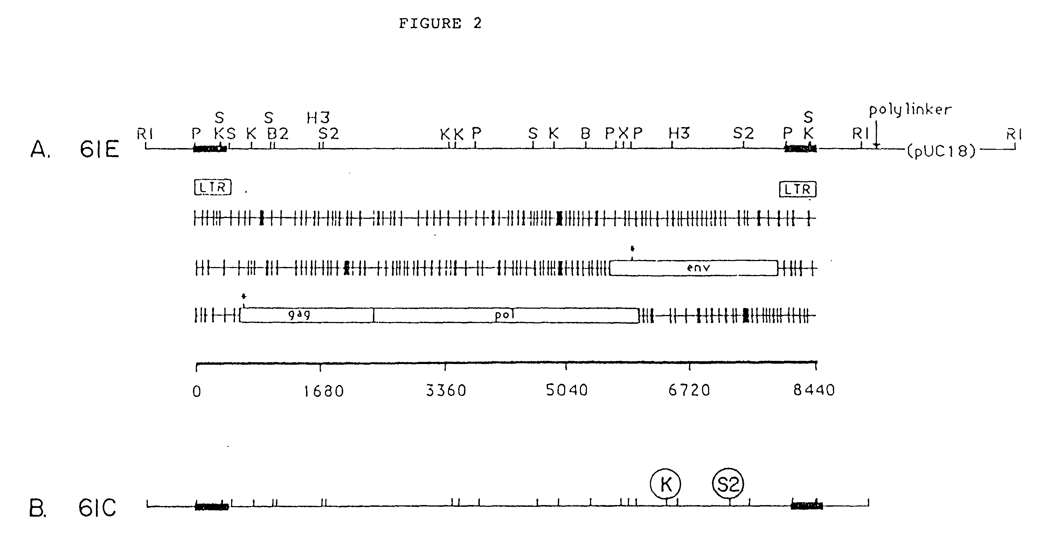

Figure 2A is a restriction site map of clone 61E with location of open reading frames (ORFs). From top to bottom frames 1 to 3 are represented. Vertical bars ( ) mark positions of termination codons. Boxes ( ) represent the ORFs known to encode viral proteins and the positions of the likely initiating methionines for the gag (gag-pol) and env translation products. Restriction sites indicated correspond to BamHI(B), BglII(B2), EcoRI(RI), HindIII(H3), KpnI(K), PstI(P), SmaI(S), SstII(S2), and XhoI(X). A restriction enzyme map of clone 61C is shown in Figure 2B. Only divergent sites are indicated in the map of the variant clone.

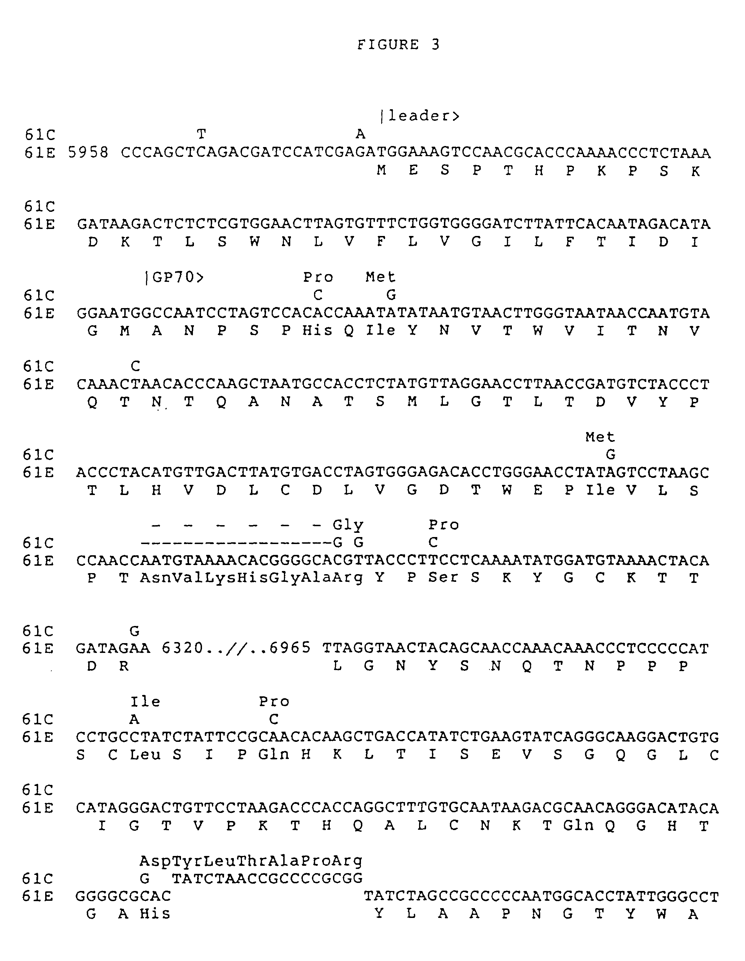

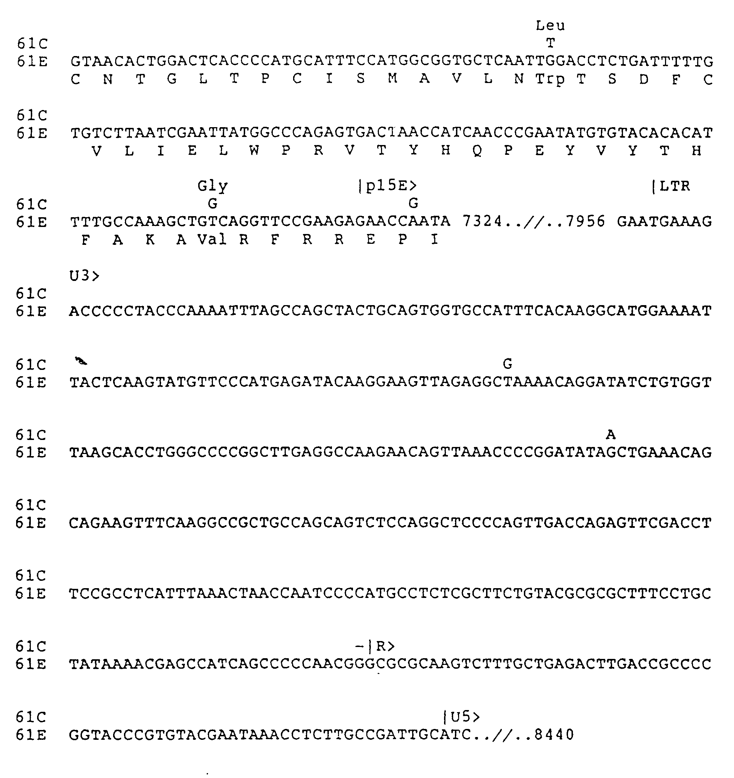

Figure 3 is depiction of the nucleotide sequence of the env-3'LTR of 61C and comparison to the corresponding region of molecular clone 61E. Portions of the nucleotide sequence of 61E are shown along with the single letter translation of the env protein. The corresponding sequence of clone 61C was determined and only those nucleotide amino acids that differ are shown here. Two stretches of complete nucleotide identity are not shown and are indicated by ..//... Where differences result in a change in the deduced amino acid sequence, the three-letter amino acid code is shown. Dashes indicate gaps.

Figure 4a is a depiction of plasmid pUC18-61E.

Figure 4b is a depiction of plasmid pUC18-61C.

Figure 4c is a depiction of plasmid pUC18-EECC.

### Detailed Description of the Invention

As noted above, the subject invention is concerned with methods and compositions for protecting feline hosts from FeLV infection, particularly by providing infectious proviral molecular clones which encode a FeLV of the A subtype or a biological derivative thereof. When certain clones, such as clone 61E, are used to transfect feline cells in vitro, such transfected cells produce infectious virus which is not cytopathic for feline T-lymphocytes or fibroblastoid cells. The growth of this virus is restricted to feline cells (and is therefore ecotropic). Virus derived from these cells is shown herein to infect specific pathogen-free cats from weanling to adult age. None of these cats has developed disease more than one year postinoculation, although they have developed persistent virus infection. Thus, the FeLV-A proviral genome encodes a prototype, ecotropic, horizontally transmissible, and minimally pathogenic feline leukemia virus. It may be distinguished from other feline leukemia viruses, in part, by its ability to consistently induce viremia in cats other than newborn age, thereby mimicking FeLV found in nature more appropriately than any other strain disclosed thus far. The nucleotide sequence of its envelope gene is very highly conserved with other strains of FeLV which are also easily transmitted and found in all FeLV-infected cats in nature. Therefore, an immune response generated to the FeLV-A based vaccines of the present invention is expected to protect against virtually any other feline leukemia virus horizontally transmissible in nature. Furthermore, because of the unique capacity of the virus encoded by DNA sequences derived from clones 61E or EECC to induce a high incidence of viremia in cats inoculated with 61E or EECC, a valid challenge system is provided which proves protection against a homologous virus challenge.

The subject invention is also concerned with methods and compositions for providing a relevant disease model in feline species that may be used to further prove an effective FeLV vaccine (i.e., protection against disease) or that may be used to study possibilities of prophylaxis and therapy of related immunodeficiencies in other species (e.g., human immunodeficiency virus infection in man). The FeLV proviral molecular clone 61C is not infectious, but, when transfected into feline cells, can be rescued by subsequent transfection of molecular clone 61E or infection with 61E virus. The resulting mixture of 61E and 61C virus, when inoculated into 8-week-old susceptible cats, induces a fatal immunodeficiency disease within four months that is typical of that observed in cats inoculated with the original FeLV-FAIDS isolate (Hoover, Blood 70:188-1892, 1987; Overbaugh, Science 239:906-910, 1988). Additionally, a chimeric FeLV proviral molecular clone may be constructed (EECC) by exchanging portions of the 61E and 61C genome. When such a construct is transfected into susceptible feline cells, the resulting virus (FeLV-EECC) is replication competent and induces immunodeficiency disease.

Within preferred embodiments for producing replication competent viruses, FeLV-61E-A and FeLV-EECC provirus are transfected into an appropriate cellular host in culture, for example, the CRFK [ATCC# CCL94] or AH927 cell line (Overbaugh, Science 239:906-910, 1988), conveniently as a provirus plasmid with or without a selectable marker. Methods of transfection may include DEAE dextran precipitation, calcium phosphate precipitation, or electroporation. As a result of the introduction of the proviral DNA, the proviral genome will become integrated into the cellular genome. The transfected cells are selected, expanded and screened for reverse transcriptase, viral production, the level of viral protein antigen released, and any other characteristics associated with the use of the virus as a vaccine.

For producing replication defective viruses, such as FeLV-61C, the proviral genome is transfected into an appropriate cellular host in culture and the resulting transformed line is cotransfected or coinfected with a replication competent proviral genome or virus, such as FeLV-61E.

Stably transfected cell lines which constitutively express FeLV protein and viruses are grown to 100% confluency in 150cm² roller bottles by seeding, for example, with a minimum of 5-6 x 10⁷ cells/roller bottle containing 250 ml cell growth medium. After cells have reached confluency in 3-7 days, the cell growth medium is discarded and the cells are replenished, for example, with 250 ml virus growth medium. The cells are further incubated, normally at 37°C, for one to several days and the FeLV containing medium is harvested. Multiple harvest, preferably a minimum of five times, is allowed until the cell monolayer begins to detach from the bottles. The FeLV harvest materials may be concentrated up to 100X depending on virus titer and/or antigenic content, as described for virus fluids. Inactivation, concentration, and adjuvanting of the cell line fluids proceeds as for virus fluids and is described herein.

Once a suitable FeLV-A subtype or biological derivative thereof has been prepared, the feline host may be inoculated by any convenient means with a sufficient amount of the inactivated virus produced from cloned proviral DNA, or with the virus and cell substrate mixtures in order provide an appropriate immune response. The amount of virus utilized will generally be from about 10⁴ to about 5 x 10⁶, usually about 1-5 x 10⁵ focus-forming units/kg host (FFU/kg). The virus may be in any convenient physiologically acceptable medium, e.g., sterile water, phosphate-buffered saline, growth medium or the like. Generally, the dosage volume will be about 0.5 to 2.0 ml, and is administered by injection subcutaneously, intramuscularly, intraperitoneally, intravenously or the like. The vaccines of the present invention may be administered to previously primed hosts. One or more booster injections may be employed at weekly to six-week, usually two- to four-week, intervals.

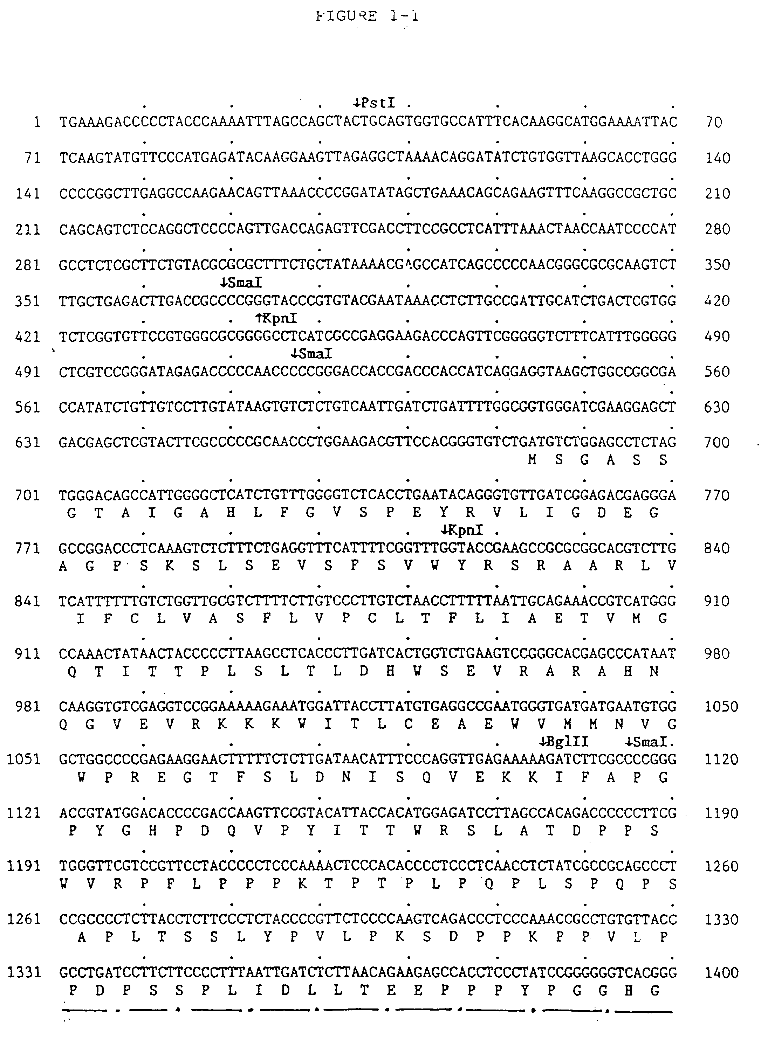

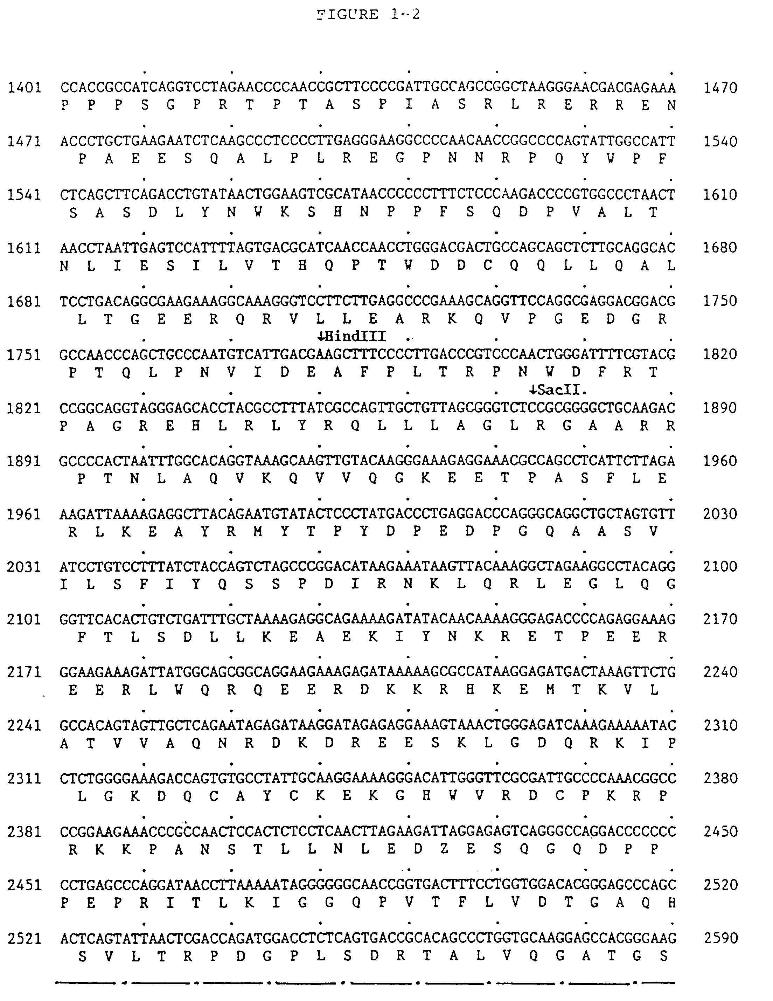

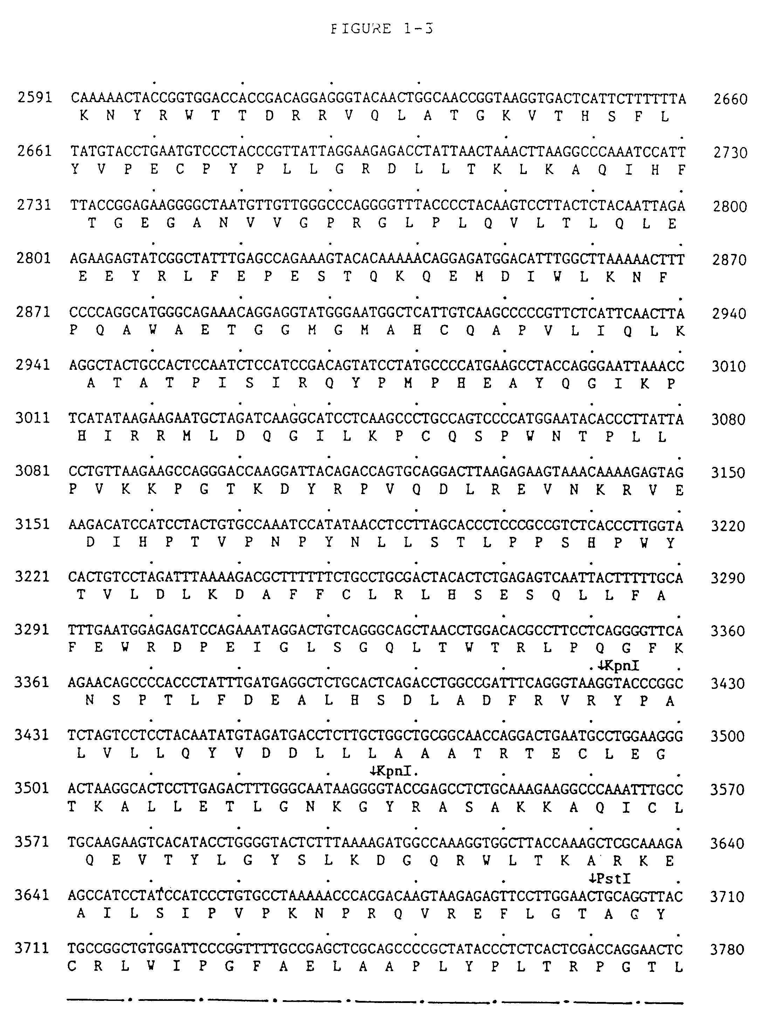

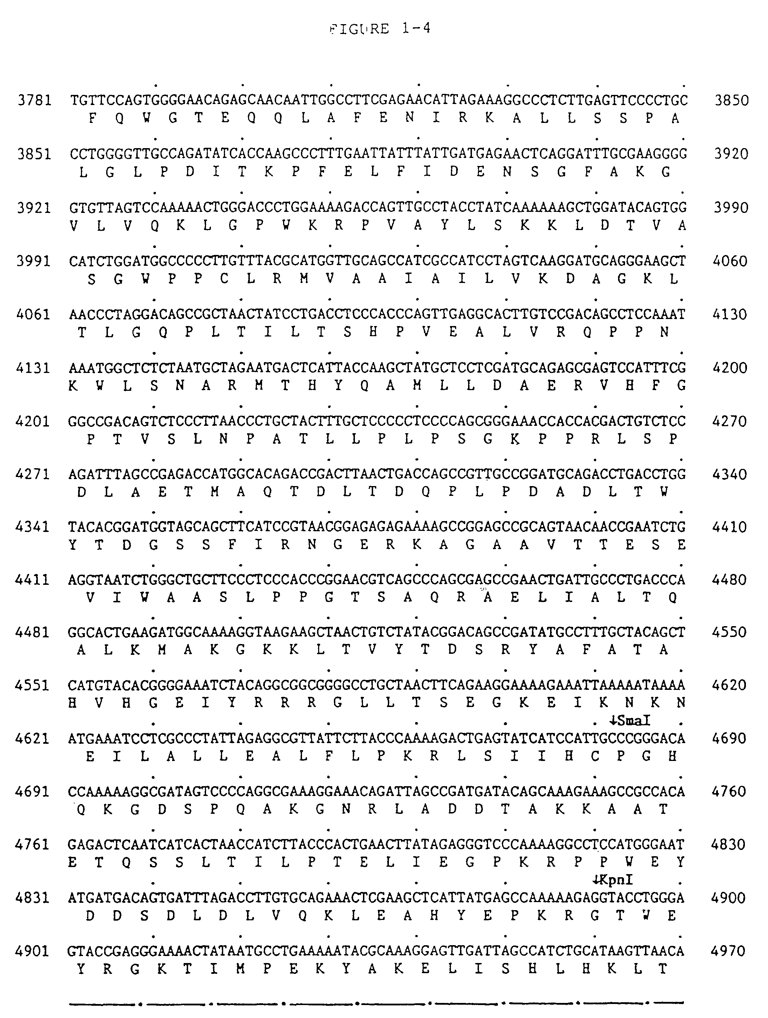

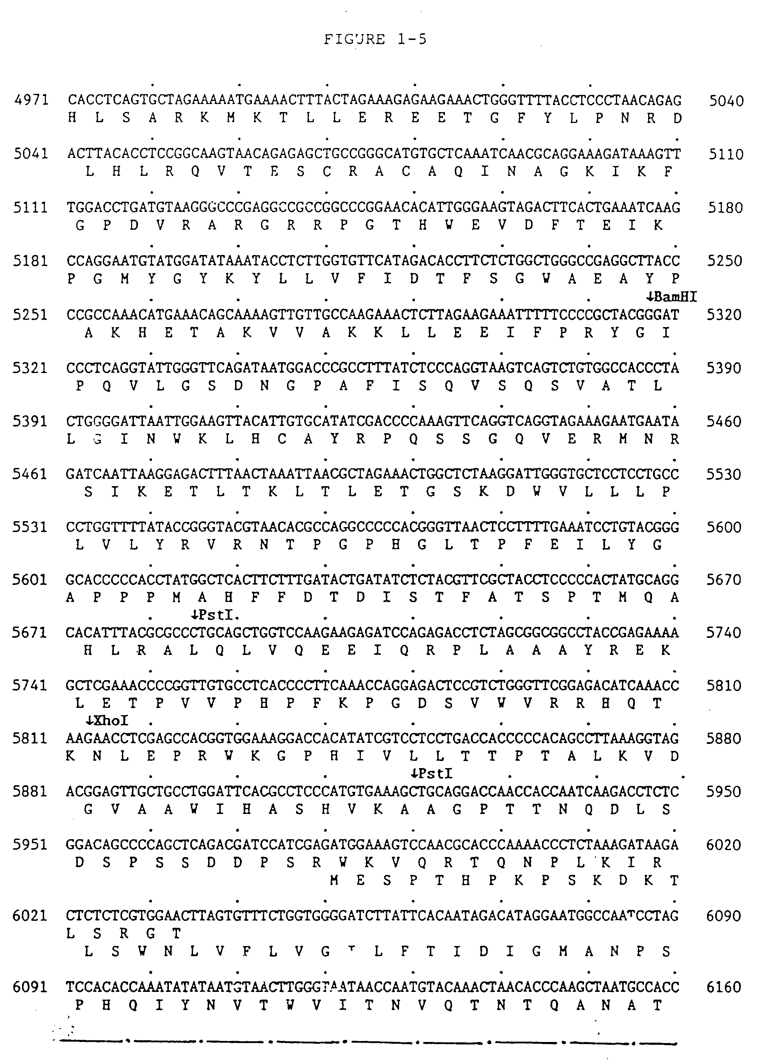

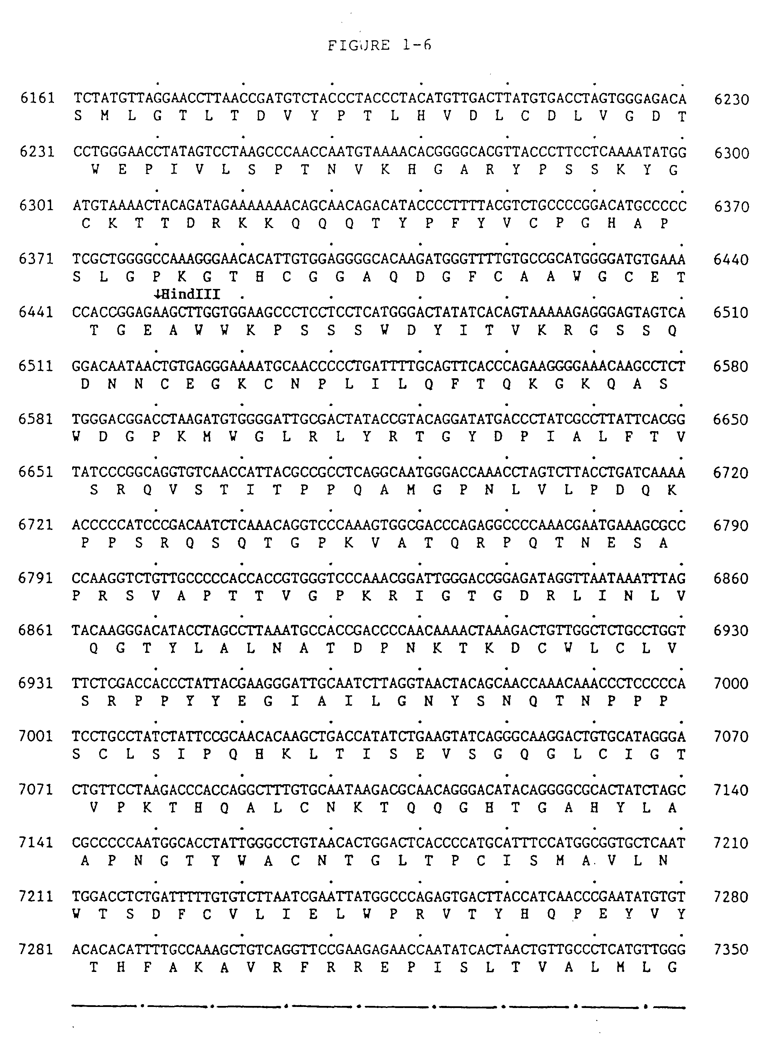

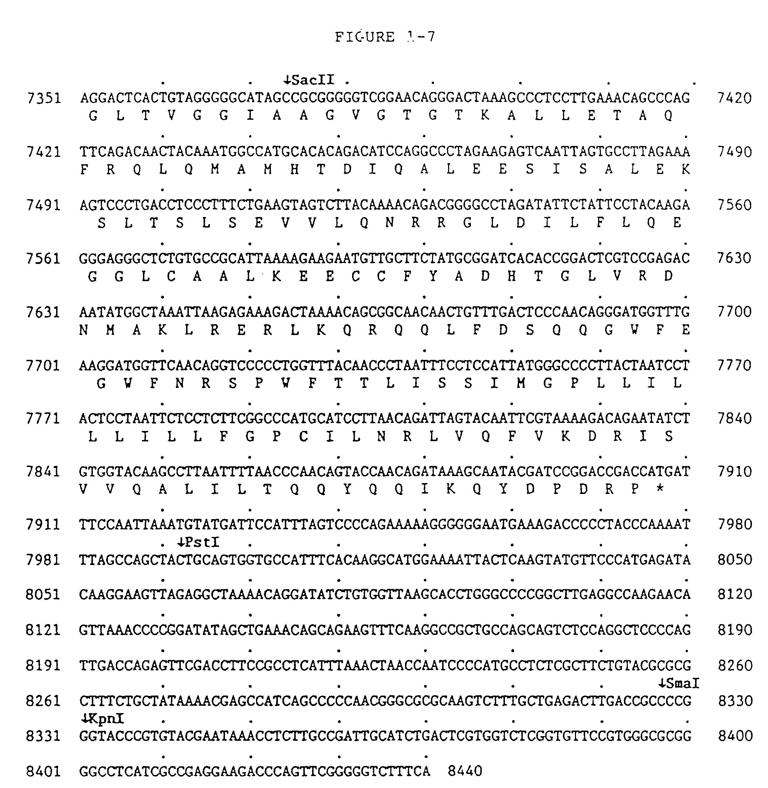

To summarize the examples which follow, the F6A virus-encoding proviral clone 61E was derived directly from intestinal tissue DNA of a specific-pathogen-free cat following inoculation with lymphosarcoma cell-free supernatant from a pet cat with a naturally occurring feline lymphosarcoma from Fort Collins, Colorado. To obtain clones with intact proviruses, DNA from cat 1161 was first cleaved with EcoRI (which does not cleave within the FeLV genome) and fractionated on a sucrose gradient. Fractions containing DNA of sufficient length to potentially contain full-length proviruses, but within the capacity of the bacteriophage vector gtWES B (P. Leder, D. Tiemeier, L. Enquist, Science 196:175, 1977), were pooled. The libraries were prepared and screened with an exogenous LTR-specific probe (Mullins et al., Nature 319:333-336, 1984). An EcoRI fragment of the full-length F6A provirus and host flanking sequences was subcloned into pUC18. Deletion clones were then generated by digestion with exonuclease BAL31, ligated into M13mp18, and sequenced by the dideoxy chain termination method. The complete 8,440 base pair (bp) sequence of the F6A provirus is shown in Figure 1 as well as the deduced amino acid sequences of the two long open reading frames. A simplified restriction map with the location of open reading frames (ORFs) corresponding to gag (encoding the nucleocapsid proteins), pol (encoding protease, reverse transcriptase, and endonuclease-integrase) and env (encoding the extracellular [gp70] and transmembrane [p15E] envelope proteins) within the F6A sequences is shown in Figure 2. The probable initiating methionines (ATG) of the gag and env genes occur at nucleotide positions 906 and 5981, respectively. In general, the F6A proviral sequences reflect a typical type C retrovirus genome. Particulars of the sequence are discussed in Donahue et al. (J. Virol. 162:722-731, 1988). Relevant to the present invention, it is significant that when the deduced gp70 protein of F6A is compared to that of two other type A isolates, F3A and FGA (Glasgow), they share remarkedly strong (98%) homology, despite their isolation from naturally infected cats up to 13 years apart and from widely separate geographic locations: FGA was isolated in Glasgow, Scotland, in 1970 and carried in culture for several years before being subjected to molecular cloning; F3A was isolated in New York City in 1977 (E. Zuckerman and W.D. Hardy, Jr., personal communication) and was also extensively propagated in culture before being cloned; and F6A was molecularly cloned from cat tissue DNA after one in vivo passage of a virus isolated from a pet cat in 1983 and was never propagated in vitro before being cloned. All three proviruses were molecularly cloned and sequenced. This remarkable sequence conservation may reflect stringent selection against antigenic changes in the ubiquitous, viremia-inducing and horizontally transmissable form of FeLV. This sequence data and comparison, together with the biological activity of F6A described below, show that F6A represents a prototype of the highly conserved, horizontally transmitted, minimally pathogenic subgroup A form of FeLV that is probably present in all naturally infected cats.

To define the biological activity of F6A, a feline embryo fibroblast cell line (AH927) was transfected with cloned 61E DNA. Reverse transcriptase (RT) was detected in the cultures by 12 days, as was proviral DNA. Sixteen 8-week old SPF cats were then inoculated with 10⁵ focus-forming units of 61E-derived F6A virus (titered by clone 81 assay, see below) derived from transfected cells. Each inoculated cat became viremic within two to four weeks and each has remained viremic since then. None has developed any signs of immunodeficiency disease after 10 months to more than 2 years. One cat developed a T⁻ cell lymphosarcoma after 21 months but the others remain healthy, indicating that F6A is minimally pathogenic, although highly infectious, and, like other chronic retroviruses, is capable of inducing lymphosarcoma with a long latency.

The following examples are offered by way of illustration and not by way of limitation.

### EXAMPLES

### EXAMPLE I

### Generation of FeLV Subtype A-Producing Cell Lines by Gene Transfer of a Molecular Clone into Susceptible Feline Cells

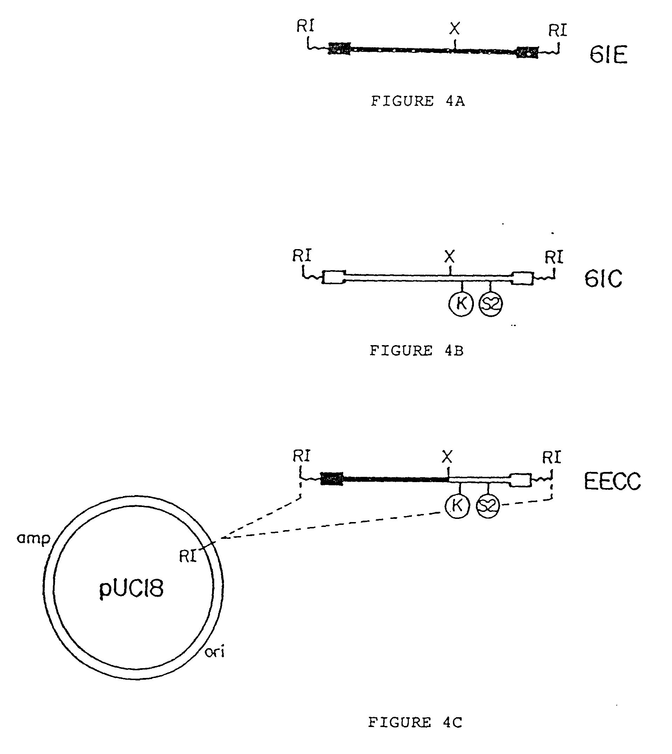

To provide for a vaccine, the FeLV-61E-A provirus was isolated as an EcoRI fragment (Figure 3) and subcloned into the EcoRI site of pUC18 (Figure 4). The resulting plasmid (pUC18-61E) was introduced into the AH927 feline embryo fibroblast cell line by transfection according to the electroporation procedure described by Potter et al. (Proc. Natl. Acad. Sci. (USA) 81:7161-7165, 1984).

The titer of infectious F6A virus released from AH927-61E was found to vary from 4-8 x 10⁶ ffu ml (Lee et al., J. Natl. Cancer Inst. 49:55-60, 1972).

### EXAMPLE II

The following description represents procedures suitable for the inactivation, concentration, and adjuvanting of cell line fluids and virus fluids within the present invention.

### Binary Ethyleneimine (BEI) Inactivation of Virus Fluids or Cell Line Fluids

Equal volumes of a 0.2 molar bromoethlamine hydrobromide solution and a 0.4 molar sodium hydroxide solution are mixed and incubated at about 37°C for 60 min. The resulting cyclized inactivant is binary ethyleneimine (BEI) which is added to the virus fluids or cell line fluids at 0.5 to 4%, volume to volume. THe inactivating virus or cell line fluids are held from 4°-37°C for 24 to 72 hr under periodic agitation.

The inactivated virus or cell line fluids are passaged three times in cell culture and examined for specific virus growth to test for complete inactivation.

### Concentration of Virus or Cell Culture Fluids

The virus or cell line fluids may be concentrated from 2 to 100 times by any number of available techniques such as Amicon, pellicon (Millipore) concentrating devices, precipitation techniques, such as ammonium chloride or polyethylene glycol (PEG), concentration with Carbowax liquid or wax in conjunction with dialysis tubing, or adjuvant concentration techniques, such as with aluminum phosphate. For the PEG concentration method, 80 ml of 50% PEG is added to 1 liter of virus or cell line fluids, then mixed overnight at 4°C. The next day the PEG-virus fluids are centrifuged at > 2500 RPM, the supernatant is discarded, and the PEG-virus pellet is resuspended in the correct volume of media to achieve the desired concentration.

### Adjuvanting Virus or Cell Culture Fluids

The following adjuvants may be used separately or in combination with 2 or more adjuvants depending on interdermal induration reactions in animals and adjuvant mixing compatability.

Ethylene maleic anhydride (EMA) prepared at a 1% weight to volume concentration in water is added to the inactivated virus or cell line fluids at 0.01% to 6% volume to volume (concentration separately or in combination with other adjuvants). The pH of the resulting fluids is adjusted to 7.1 to 7.7 by addition of 1 N sodium hydroxide.

Neocryl A640 is a trade name for a latex emulsion of a copolymer (A or styrene and a mixture of acrylic acid and methacrylic acid). Neocryl A640 is an uncoalesced aqueous acrylic copolymer with styrene, having pH 7.5, viscosity 100 cps (Brookfiled 25°C), weight per gallon is 8.6 lbs as supplied containing 40% solids by weight and 38% solids by volume. The numeral A640 denotes a grade thereof. Other useful Neocryl grades are 520, 625, and 966. The term "CSMA" is used hereinafter to refer to a copolymer of styrene and a mixture of acrylic acid and methacrylic acid. CSMA prepared in a 50% volume per volume suspension in water is added to the inactivated virus or cell line fluids from 0.2 to 10% volume separately or in combination with other adjuvants. Usually there is no need for pH adjustment since the CSMA is a neutral pH.

Modern Veterinary Products (Omaha, Nebr.) Emulsigen adjuvant for small animals is an oil-in-water emulsion which is used separately or in combination with other adjuvants in a 1 to 20% volume to volume of virus or cell line fluids.

Avridine is used separately or in combination with other adjuvants at from 5 to 30 mg per dose. Avridine at 2.4 gm is dissolved in 18 ml of absolute ethyl alcohol, then 1.8 ml of Tween-80 is added and the mixture is passed through a 0.2 micron filter. Subsequently, 20.2 ml of Intralipid soy bean oil is asceptically added to the avridine. Seven to 50% of this adjuvant is then added volume to volume to the virus or cell line fluids.

Raw or purified saponin is used separately or in combination with other adjuvants at from 0.05 mg to 5 mg per dose. Saponin is prepared at a 200 mg/ml concentration, filter sterilized and then added to the virus or cell line fluids at from 0.05 to 20% volume to volume.

Aluminum phosphate at from .01 to 5 mg per dose or aluminum hydroxide at from 0.5 to 20 mg per dose may also be used separately or in combination with other adjuvants.

### Cell and Virus Growth Medium

In vaccine production, cells may be grown in minimal essential media (MEM) supplemented with vitamins, nonessential amino acids, sodium pyruvate, sodium bicarbonate (Gibco, Grand Island, NY) containing 10-25 mM Hepes buffer, 30 g/ml polymyxin and 30 g/ml neomycin which has been filter sterilized and stored at 4C. Prior to addition to cells, 2 mM L-glutamine and bovine serum is added to the medium. For cell growth, the bovine serum is added to 10%. For cell maintenance, the bovine serum is added to 0.05%. The average harvest is preferably greater than 10⁴ particles/ml.

### EXAMPLE III

### Use of F6A Virus from the AH927-61E Cell Line as a Vaccine Utilizing Freund's Adjuvant

For purposes of vaccine preparation, AH927-61E cells were grown as follows. F6A virus containing cell supernatants (2 x 10⁵ ffu/ml) were collected, in some instances concentrated, and inactivated with 0.1%-2% formalin or binary ethylene imine (BEI). Inactivated viral preparations were formulated with complete or incomplete Freund's adjuvant and delivered to SPF cats in 1 ml (?) intramuscular inoculations.

For the studies described in Table 1, at the start of vaccination, cats ranged in age from 4 to 10 months. Two to three doses of vaccine were delivered in the time interval described. Control immunizations included the Norden LEUKOCELL vaccine or adjuvant only. Cats were challenged with either F6A virus from AH927-61E (10⁵-10⁶ ffu administered intraperitoneally) or FeLV CSU field isolate virus #05821 (10⁵ ffu delivered intranasally). The challenge was or was not accompanied by treatment of the cats with corticosteroids as noted in Table 1. Cats were bled from the jugular vein post challenge and assessed for FeLV viremia by p27 ELISA antigen detection in the serum and by immunofluorescence in blood cells. Cats with persistent viremia are considered to have been successfully infected by the challenge. Cats resisting persistent viremia are considered uninfected, i.e., to have resisted challenge. When cats are immunosuppressed, 75%-83% of control animals develop persistent viremia after challenge. Without immunosuppression, 60% of animals develop viremia. In subsequent studies, doses of challenge virus have been increased in order to produce 100% viremia in control animals. Only 5% of all 61E immunized animals developed persistent viremia. In one study (CSUJJ4), 100% of 61E cats resisted viremia from challenge with a heterologous FeLV isolate (05821) of the AB subtype, while only 25% of control cats resisted the challenge. This confirms the ability of the 61E-based vaccine preparation to protect against a heterologous challenge such as would occur in nature. In study CSUFD1, only two inoculations were adequate to protect 100% of animals from viremia (60% of control animals viremic).

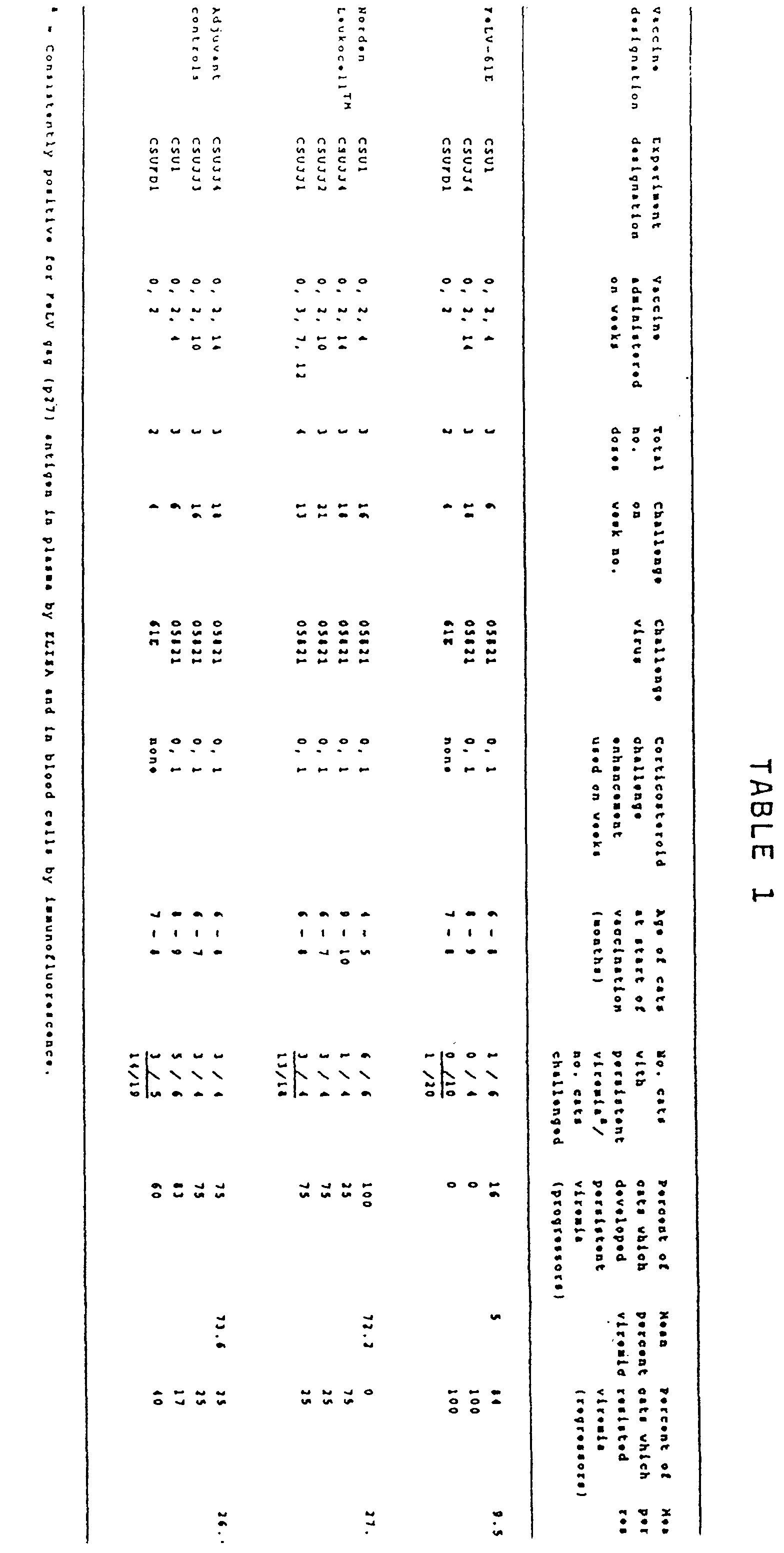

### EXAMPLE IV

### Generation of FoLV-Induced Disease Model

In the intestinal DNA preparation from which molecular clone 61E was isolated, an equal copy number of variant genomes (as defined by restriction enzyme polymorphism; see Figure 2) was noted. A molecular clone corresponding to a prototype of the "variant A" genome referred to as 61C was isolated in a bacteriophage vector and subsequently inserted in a plasmid vector (Figure 4b). The plasmid was used to transfect feline fibroblast and T-lymphocyte cultures in vitro. The sequence failed to encode infectious virus. When cotransfected with pFeLV-61E, FeLV-61C was highly infectious and the resulting mixture was cytopathic for T-lymphocytes.

A chimeric virus was constructed in vitro between pFeLV-61E and pFeLV-61C, exchanging sequences on either side of the unique Xho I restriction site found in each provirus at approximately nucleotide position 5817. The 5' terminal sequences were derived from pFeLV-61E, and the 3' sequences were derived from FeLV-61C. The plasmid referred to as pFeLV-EECC (Figure 4c) was used to transfect feline T-lymphocyte cultures in vitro and shown to encode infectious cytopathic virus.

Both the FeLV-61E/61C virus mixture and FeLV-EECC have been shown to induce fatal immunodeficiency disease (Hoover, Blood 70:1880-1892, 1987; Overbaugh, Science 239:906-910, 1988) in all inoculated and persistently viremic cats. The replication-competent FeLV-EECC and similar chimeras induce shorter latency disease, with survival times ranging from 20 to 60 days following inoculation of weanling cats. Survival times for animals inoculated with the FeLV-61E/61C mixture range from 90-120 days.

The nucleotide sequence of the portion of the 61C genome found in the FeLV-EECC chimera was determined and the divergent sequences relative to FeLV-61E identified (Figure 3). The mixture and chimera are the first fully defined retroviruses shown to induce fatal immunodeficiency disease in any organism. They may therefore be used for identification of genetic sequences responsible for AIDS induction in cats, evaluation of anti-viral drug efficacy, as challenge viruses for evaluation of the efficacy of vaccines in preventing viremia and disease, and in the development of vaccines to stimulate immunity against feline leukemia viruses.

Although the foregoing invention has been described in some detail by way of illustration and example for purposes of clarity of understanding, it will be obvious that certain changes and modifications may be practiced within the scope of the appended claims.

The features disclosed in the foregoing descriptio in the claims and/or in the accompanying drawings may, both, separately and in any combination thereof, be material for realising the invention in diverse forms thereof.

## Claims

1. A method of producing a FeLV vaccine, which method comprises transfecting a mammalian host cell with a molecular clone capable of directing the expression of the proviral genome of a FeLV-A subtype or a biological derivative thereof, said molecular clone comprising a DNA sequence encoding the proviral genome of a FeLV-A subtype or a biological derivative thereof, growing said host cell in an appropriate medium, harvesting the FeLV-A subtype or biological derivative immunogens, and inactivating the FeLV-A subtype or biological derivative thereof.

2. A method of producing a FeLV vaccine, which method comprises transfecting a mammalian host cell with a molecular clone capable of directing the expression of the proviral genome of a FeLV-A subtype or a biological derivative thereof, said molecular clone comprising a DNA sequence derived from the clone 61C, clone EECC or clone 61E and a DNA sequence derived from a FeLV clone which is replication competent, growing said host cell in an appropriate medium, harvesting the FeLV-A subtype or biological derivative immunogens, and inactivating the FeLV-A subtype or biological derivative thereof.

3. A method of producing a FeLV vaccine, which method comprises co-transfecting a mammalian host cell with a first molecular clone capable of directing the expression of the proviral genome of a FeLV-A subtype or a biological derivative thereof, said first molecular clone comprising a DNA sequence derived from clone 61C, and with a second molecular clone capable of directing the expression of the proviral genome of a FeLV-A subtype or a biological derivative thereof, said second molecular clone comprising a DNA sequence derived from a FeLV clone which is replication competent, growing said host cell in an appropriate medium, harvesting the FeLV-A subtype or biological derivative immunogens, and inactivating the FeLV-A subtype or biological derivative thereof.

4. A composition comprising an inactivated FeLV-A subtype as the sole FeLV subtype or a biological derivative thereof, producible by a method according to Claim 1 or 2, in combination with a physiologically acceptable carrier or diluent.

5. A composition according to Claim 4, wherein the composition includes a suitable adjuvant.

6. A vaccine against FeLV-induced disease, comprising an inactived FeLV-A subtype as the sole FeLV subtype or a biological derivative thereof producible by a method according to Claim 1 or 2, in combination with a suitable adjuvant.

7. A vaccine according to Claim 6, wherein said FeLV-A subtype is encoded by a DNA sequence derived from clone 61E.

## Patentansprüche

1. Verfahren zur Herstellung eines FeLV-Impfstoffs, wobei das Verfahren umfaßt: Transfizieren einer Säugetierwirtszelle mit einem molekularen Klon, der in der Lage ist, die Expression des proviralen Genoms eines FeLV-A-Subtyps oder eines biologischen Derivats davon zu steuern, wobei besagter molekularer Klon eine DNA-Sequenz umfaßt, die für das provirale Genom eines FeLV-A-Subtyps oder eines biologischen Derivats davon kodiert, Wachsenlassen von besagter Wirtszelle in einem geeigneten Medium, Ernten der FeLV-A-Subtyp- oder biologischen-Derivat-Immunogene, und Inaktivieren des FeLV-A-Subtyps oder eines biologischen Derivats davon.

2. Verfahren zur Herstellung eines FeLV-Impfstoffs, wobei das Verfahren umfaßt: Transfizieren einer Säugetierwirtszelle mit einem molekularen Klon, der in der Lage ist, die Expression des proviralen Genoms eines FeLV-A-Subtyps oder eines biologischen Derivats davon zu steuern, wobei besagter molekularer Klon eine DNA-Sequenz, die aus dem Klon 61C, Klon EECC oder Klon 61E stammt, und eine DNA-Sequenz umfaßt, die aus einem FeLV-Klon stammt, der replikationskompetent ist, Wachsenlassen von besagter Wirtszelle in einem geeigneten Medium, Ernten der FeLV-A-Subtyp- oder biologischen-Derivat-Immunogene und Inaktivieren des FeLV-A-Subtyps oder eines biologischen Derivats davon.

3. Verfahren zur Herstellung eines FeLV-Impfstoffs, wobei das Verfahren umfaßt: Ko-Transfizieren einer Säugetierwirtszelle mit einem ersten molekularen Klon, der in der Lage ist, die Expression des proviralen Genoms eines FeLV-A-Subtyps oder eines biologischen Derivats davon zu steuern, wobei besagter erster molekularer Klon eine DNA-Sequenz, die aus Klon 61C stammt, umfaßt, und mit einem zweiten molekularen Klon, der in der Lage ist, die Expression des proviralen Genoms eines FeLV-A-Subtyps oder eines biologischen Derivats davon zu steuern, wobei besagter zweiter molekularer Klon eine DNA-Sequenz umfaßt, die aus einem FeLV-Klon stammt, der replikationskompetent ist, Wachsenlassen besagter Wirtszelle in einem geeigneten Medium, Ernten der FeLV-A-Subtyp- oder biologischen-Derivat-Immunogene, und Inaktivieren des FeLV-A-Subtyps oder biologischen Derivats.

4. Zusammensetzung, umfassend einen inaktivierten FeLV-A-Subtyp als den einzigen FeLV-Subtyp oder ein biologisches Derivat davon, herstellbar durch ein Verfahren gemäß Anspruch 1 oder 2, in Kombination mit einem physiologisch annehmbaren Träger oder Verdünnungsmittel.

5. Zusammensetzung nach Anspruch 4, wobei die Zusammensetzung ein geeignetes Adjuvans einschließt.

6. Impfstoff gegen FeLV-induzierte Krankheit, umfassend einen inaktivierten FeLV-A-Subtyp als den einzigen FeLV-Subtyp oder ein biologisches Derivat davon, herstellbar durch ein Verfahren gemäß Anspruch 1 oder 2, in Kombination mit einem geeigneten Adjuvans.

7. Impfstoff gemäß Anspruch 6, wobei besagter FeLV-A-Subtyp von einer DNA-Sequenz kodiert wird, die aus Klon 61E stammt.

## Revendications

1. Procédé pour la production d'un vaccin contre le FeLV, procédé qui comprend les étapes consistant à transfecter une cellule hôte de mammifère avec un clone moléculaire capable de diriger l'expression du génome proviral d'un sous-type FeL-A ou d'un de ses dérivés biologiques, ledit clone moléculaire comprenant une séquence d'ADN codant pour le gènome proviral d'un sous-type FeLV-A ou d'un de ses dérivés biologiques, à cultiver ladite cellule hôte dans un milieu approprié, à recueillir les agents immunogènes consistant en le sous-type FeLV-A ou ses dérivés biologiques, et inactiver le sous-type FeLV-A ou son dérivé biologique.

2. Procédé pour la production d'un vaccin contre le FeLV, procédé qui comprend les étapes consistant à transfecter une cellule hôte de mammifère avec un clone moléculaire capable de diriger l'expression du génome proviral d'un sous-type FeLV-A ou d'un de ses dérivés biologiques, ledit clone moléculaire comprenant une séquence d'ADN dérivée du clone 61C, du clone EECC ou du clone 61E et une séquence d'ADN dérivée d'un clone de FeLV qui est compétent du point de vue de la réplication, à cultiver ladite cellule hôte dans un milieu approprié, à recueillir les agents immunogènes consistant en le sous-type FeLV-A ou ses dérivés biologiques, et à inactiver le sous-type FeLV-A ou son dérivé biologique.

3. Procédé pour la production d'un vaccin contre le FeLV, procédé qui comprend les étapes consistant à cotransfecter une cellule hôte de mammifère avec un premier clone moléculaire capable de diriger l'expression du génome proviral d'un sous-type FeLV-A ou d'un de ses dérivés biologiques, ledit premier clone moléculaire comprenant une séquence d'ADN dérivée du clone 61C, et avec un second clone moléculaire capable de diriger l'expression du génome proviral d'un sous-type FeLV-A ou d'un de ses dérivés biologiques, ledit second clone moléculaire comprenant une séquence d'ADN dérivée d'un clone de FeLV qui est compétent du point de vue de la replication, à cultiver ladite cellule hôte dans un milieu approprié, à recueillir les agents immunogènes consistant en le sous-type FeLV-A ou ses dérivés biologiques, et à inactiver le sous-type FeLV-A ou son dérivé biologique.

4. Composition comprenant un sous-type FeLV-A inactivé comme seul sous-type de FeLV ou un de ses dérivés biologiques, pouvant être produit par un procédé suivant la revendication 1 ou 2, en association avec un support ou diluant physiologiquement acceptable.

5. Composition suivant la revendication 4, qui comprend un adjuvant convenable.

6. Vaccin contre une maladie induite par le FeLV, comprenant un sous-type FeLV-A inactivé comme seul sous-type de FeLV ou un de ses dérivés biologiques, pouvant être produit par un procédé suivant la revendication 1 ou 2, en association avec un adjuvant convenable.

7. Vaccin suivant la revendication 6, dans lequel le sous-type FeLV-A est codé par une séquence d'ADN dérivée du clone 61E.
